(19) Europäisches Patentamt / European Patent Office / Office européen des brevets

(11) EP 3 146 889 A1

(12) EUROPEAN PATENT APPLICATION
published in accordance with Art. 153(4) EPC

(43) Date of publication:
29.03.2017 Bulletin 2017/13

(51) Int Cl.:
A61B 1/06 (2006.01)  A61B 1/00 (2006.01)
A61B 1/04 (2006.01)  F21S 2/00 (2016.01)
G03B 7/16 (2014.01)  G03B 15/02 (2006.01)
G03B 15/05 (2006.01)  H04N 9/04 (2006.01)
H05B 37/02 (2006.01)

(21) Application number: 15792206.3

(22) Date of filing: 26.03.2015

(86) International application number:
PCT/JP2015/059479

(87) International publication number:
WO 2015/174146 (19.11.2015 Gazette 2015/46)

(84) Designated Contracting States:
AL AT BE BG CH CY CZ DE DK EE ES FI FR GB
GR HR HU IE IS IT LI LT LU LV MC MK MT NL NO
PL PT RO RS SE SI SK SM TR
Designated Extension States:
BA ME
Designated Validation States:
MA

(30) Priority: 14.05.2014 JP 2014100797

(71) Applicant: Olympus Corporation
Tokyo 192-8507 (JP)

(72) Inventor: GODO Hirokazu
Hachioji-shi
Tokyo 192-8507 (JP)

(74) Representative: Gunzelmann, Rainer
Wuesthoff & Wuesthoff
Patentanwälte PartG mbB
Schweigerstraße 2
81541 München (DE)

(54) IMAGE PICKUP SYSTEM

(57) An image pickup system includes a first LED (14) whose first light emission quantity decreases as a first cumulative lighting time period increases, a second LED (15) whose second light emission quantity decreases as a second cumulative lighting time period increases, an image pickup device (22) configured to pick up an image of an object irradiated with light from the first and second LEDs (14, 15) of which colors are different from each other and generate first and second video signals, and a light-emitting element degradation level correction section in a video signal processing section (17) configured to acquire the first and second light emission quantities based on the first and second cumulative lighting time periods, perform gain adjustment based on a light emission quantity ratio on the first and second video signals and thereby correct a difference in a degradation level between the first and second LEDs (14, 15).

FIG. 1

EP 3 146 889 A1

**Description**

Technical Field

The present invention relates to an image pickup system that causes a plurality of light-emitting elements in different bands to emit light, illuminates an object and picks up an image of the object.

Background Art

Image pickup systems configured to illuminate an object and pick up an image of the object are applied to various fields such as digital cameras and digital video cameras, and the field of endoscopes is an example of fields where there is substantially no ambient light and illuminating light is essential.

As a light source of illuminating light for such endoscopes, light-emitting elements such as LEDs having an advantage of high light emission efficiency are starting to be used in recent years. When LEDs are used as a light source, a plurality of color LEDs to acquire a color image are required and the light source is configured so as to include an R-LED, a G-LED and a B-LED, for example.

These respective color LEDs may degrade as the operating time (cumulative lighting time period) increases and the light quantity may decrease, and in addition, the degradation level may vary from one color to another. For this reason, even when the ratio of light emission quantities among the respective color LEDs in an initial state (state of unused new product) is adjusted so that white light is configured, the color balance may deviate from white color as the cumulative lighting time period increases. Various techniques are conventionally proposed as techniques for correcting deviation in color balance accompanying the degradation of light-emitting elements.

For example, Japanese Patent Application Laid-Open Publication No. 2012-32619 describes a technique that provides R, G and B LEDs as light sources and corrects deviations of color balance in illuminating light caused by differences in degradation level over time among the color LEDs by changing a value of current supplied to each LED. To be more specific, the Publication describes that assuming that actual light emission color temperature - set light emission color temperature = difference value data, average difference value data is calculated from a plurality of pieces of difference value data to suppress the variations, a forward direction current that flows through each LED is adjusted when the average difference value data becomes equal to or above a correction threshold (±100K) to correct the light emission quantity. Furthermore, the Publication also describes a technique that when a set light emission color temperature is low, the light emission quantity of the R-LED needs to be increased, which leads to degradation of the R-LED, and when the set light emission color temperature is high, the light emission quantity of the BLED needs to be increased, which leads to degradation of the B-LED, and therefore the color temperature is divided into a plurality of regions and average difference value data is calculated to thereby improve correction accuracy.

Furthermore, Japanese Patent Application Laid-Open Publication No. 2011-233263 describes a technique that counts a cumulative lighting time period of an LED and calculates an evaluation value indicating the degradation level of the LED based on the counted cumulative lighting time period to deal with the degradation of the LED as the lighting time period increases. More specifically, the technique described in the Publication also takes into consideration the fact that when the lighting time period remains unchanged, the higher the light adjustment rate becomes, the greater the degradation becomes, assumes the total value of products of the lighting time period and the light adjustment rate to be an evaluation value, thereby estimates a total value of current (or power) that has flown through the LED so far and eventually estimates the degradation level of the LED. The evaluation value indicating the degradation level is stored in a storage section and is used when LEDs with less degradation are used with high priority. It is thereby possible to substantially equalize frequencies of use of a plurality of LEDs so that the plurality of LEDs have the same life cycle.

Furthermore, International Publication No. WO2010/041494 describes that with an illumination apparatus of a liquid crystal display device, an R-LED, a G-LED and a B-LED for generating white light gradually degrade as the operating time increases, but since the degradation rate varies among the LEDs, color balance is lost. In order to prevent the color balance from varying depending on the difference in the degradation rate of each color LED, a coloration correction operation is performed, the degradation rate of each color LED is calculated from the output value of a photodiode obtained by causing each color LED to emit light and the value of current supply to each color LED is thereby adjusted based on the calculated degradation rate. The amount of current supply at this time is calculated by fixing luminous intensity of the LED having the highest degradation level, and reducing and adjusting luminous intensities of other LEDs.

As described above, when, for example, white illumination is performed using LEDs in a plurality of colors, since the degradation rate varies from one LED color to another, the color balance varies due to a secular change. The technique described in Japanese Patent Application Laid-Open Publication No. 2012-32619 has a difficulty in accurately measuring color temperatures. The technique in International Publication No. WO2010/041494 requires a dedicated sensor such as a photodiode to calculate the degradation rate of the LED. In contrast to these techniques, the technique described in Japanese Patent Application Laid-Open Publication No. 2011-233263 calculates the degradation level of the LED based on the cumulative lighting time period, but the calculated degradation level is used to equalize the life cycles of the LEDs and not used to correct the color balance. The techniques described in Japanese Patent Application Laid-Open Publication No. 2012-32619 and International Publication No. WO2010/041494 adjust the value of current supply to the respective color LEDs to thereby adjust the color balance, but in this case, a control circuit capable of fine-tuning the current value is necessary, which leads to a cost increase.

The present invention has been implemented in view of the above-described circumstances and it is an object of the present invention to provide an image pickup system capable of preventing, at low cost, a color balance of illuminating light from changing with time.

Disclosure of Invention

Means for Solving the Problem

An image pickup system according to an aspect of the present invention is an image pickup system that irradiates an object with light and picks up an image of the object, including a first light-emitting element configured to turn on when power is supplied to emit first-band light, a first light emission quantity of which decreases at a first degradation rate as a first cumulative lighting time period increases, a second light-emitting element configured to turn on when power is supplied to emit second-band light, a second light emission quantity of which decreases at a second degradation rate as a second cumulative lighting time period increases, an image pickup section configured to pick up an optical image of the object irradiated with the light from the first light-emitting element and the second light-emitting element and generate a first video signal associated with the first-band light and a second video signal associated with the second-band light, and a degradation level correction section configured to acquire the first light emission quantity based on the first cumulative lighting time period, acquire the second light emission quantity based on the second cumulative lighting time period, calculate a gain coefficient by dividing one of the first light emission quantity and the second light emission quantity by another, perform gain adjustment using the calculated gain coefficient on another of the first video signal and the second video signal and thereby correct a difference in a degradation level between the first light-emitting element and the second light-emitting element.

Brief Description of the Drawings

Fig. 1 is a block diagram illustrating a configuration of an image pickup system according to Embodiment 1 of the present invention;

Fig. 2 is a block diagram illustrating a configuration of a video signal processing section according to Embodiment 1 of the present invention;
Fig. 3 is a time chart illustrating an example of a light emission quantity attenuation ratio of each color LED due to a change over time according to Embodiment 1 of the present invention;
Fig. 4 is a time chart illustrating an example of a case where light emission quantity attenuation ratios due to a change over time become substantially equal for respective color LEDs according to Embodiment 1 of the present invention;
Fig. 5 is a diagram illustrating a configuration of an LED drive circuit associated with Embodiment 1 of the present invention;
Fig. 6 is a timing chart illustrating an example of a signal flowing through the LED drive circuit in Fig. 5 associated with Embodiment 1 of the present invention; and
Fig. 7 is a timing chart illustrating an example where a current waveform becomes unstable when a plurality of LEDs are caused to emit light by one LED driver associated with Embodiment 1 of the present invention.

Best Mode for Carrying Out the Invention

Hereinafter, an embodiment of the present invention will be described with reference to the accompanying drawings.

[Embodiment 1]

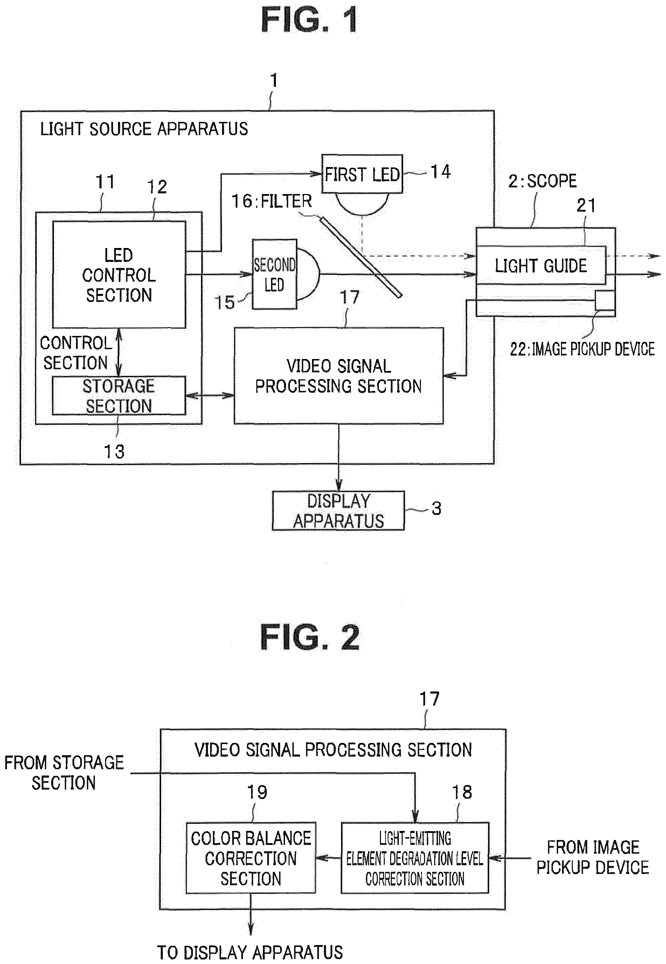

Fig. 1 to Fig. 4 illustrate Embodiment 1 of the present invention and Fig. 1 is a block diagram illustrating a configuration of an image pickup system.
As shown in Fig. 1, the image pickup system is a system that illuminates an object and picks up an image of the object, and includes a scope 2 as an image pickup apparatus, a light source apparatus 1 configured to supply illuminating light to the scope 2 and process a video signal outputted from the scope 2, and a display apparatus 3 configured to display the video signal processed by the light source apparatus 1.
First, the scope 2 is configured as, for example, an electronic endoscope and is provided with a light guide 21 configured to transmit the illuminating light supplied from the light source apparatus 1 to a distal end side so that the illuminating light is radiated onto the object, and an image pickup device 22 such as a CCD or a CMOS configured to pick up an optical image of the object irradiated with the illuminating light and generate the video signal.
Here, the image pickup device 22 is an image pickup section configured to pick up an optical image of the object illuminated by a first LED 14, which will be described later, of the light source apparatus 1, generate a first video signal associated with first-band light, and pick up an optical image of the object illuminated by a second LED 15, which will be described later, of the light source apparatus 1, and generate a second video signal associated with second-band light. To be more specific, the image pickup device 22 is configured as, for example, a single-chip color image pickup device and enabled to generate an R video signal associated with red (R)-band light, a G video signal associated with green (G)-band light and a B video signal associated with blue (B)-band light. Note that when violet (V)-band light is included as the illuminating light supplied from the light source apparatus 1 (the violet (V)-band light is used, for example, in an NBI (registered trademark) (narrow band imaging) observation mode or a fluorescence observation mode), the image pickup device 22 generates a V video signal associated with the violet (V)-band light using pixels to receive, for example, the blue (B)-band light.

Next, the light source apparatus 1 is provided with a control section 11 including an LED control section 12 and a storage section 13, a first LED 14, a second LED 15, a filter 16 and a video signal processing section 17.

The control section 11 is configured to include, for example, a CPU and control the light source apparatus 1.

The LED control section 12 switches whether or not to supply power to turn on/off the first LED 14 and the second LED 15 respectively and controls power to be supplied to the first LED 14 and the second LED 15 through current adjustment, PWM adjustment or a combination of these adjustments to thereby control respective light emission quantities of the first LED 14 and the second LED 15. The control by the LED control section 12 over the first LED 14 and the second LED 15 is performed according to an observation mode such as a white light observation mode, an NBI observation mode and a fluorescence observation mode.

The storage section 13 stores a first degradation rate (the degradation rate here is a degradation rate with respect to a unit cumulative lighting time period) of the first LED 14, a second degradation rate of the second LED 15, a first initial light emission quantity of the first LED 14 in an initial state (state of a new unused product) and a second initial light emission quantity of the second LED 15 in an initial state. Furthermore, the storage section 13 receives a first cumulative lighting time period of the first LED 14 and a second cumulative lighting time period of the second LED 15 from the LED control section 12 and stores those cumulative lighting time periods.

Note that when the first light emission quantity which is the current light emission quantity of the first LED 14 and the second light emission quantity which is the current light emission quantity of the second LED 15 are acquired by referring to a table instead of carrying out calculations, the storage section 13 stores a first table showing a correspondence between the first cumulative lighting time period and the first light emission quantity (this correspondence corresponds to the aforementioned first degradation rate) and a second table showing a correspondence between the second cumulative lighting time period and the second light emission quantity (this correspondence corresponds to the aforementioned second degradation rate).

The first LED 14 is a first light-emitting element configured to turn on when power is supplied under the control of the LED control section 12 and emit first-band light whose light emission quantity decreases at a first degradation rate as the first cumulative lighting time period increases.

The second LED 15 is a second light-emitting element configured to turn on when power is supplied under the control of the LED control section 12 and emit second-band light whose light emission quantity decreases at a second degradation rate as the second cumulative lighting time period increases.

Here, the first degradation rate may be different from the second degradation rate or may be the same as the second degradation rate.

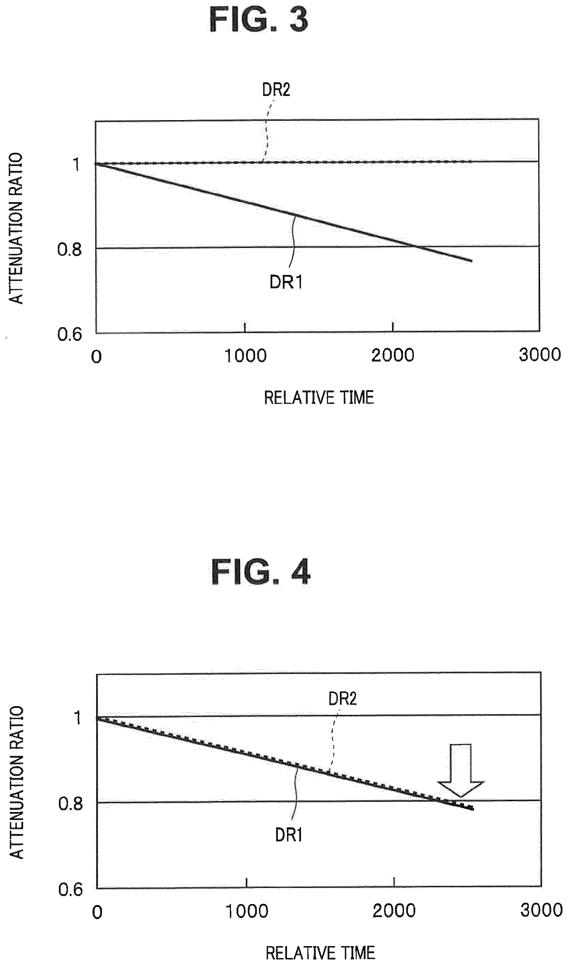

With reference to Fig. 3, a case where the first degradation rate is different from the second degradation rate will be described. Fig. 3 is a time chart showing an example of a light emission quantity attenuation ratio of each color LED due to a change over time. This Fig. 3 (and Fig. 4 which will be described later) shows an attenuation ratio of light emission quantity as a relative cumulative lighting time period increases when the light emission quantities of the first LED 14 and the second LED 15 at a cumulative lighting time period of 0 are assumed to be 1.

In the example shown in Fig. 3, an attenuation ratio DR2 of the second LED 15 shows substantially no decrease even when the relative cumulative lighting time period increases.

In contrast, in the example shown in Fig. 3, an attenuation ratio DR1 of the first LED 14 decreases at a higher rate than the attenuation ratio DR2 of the second LED 15 as the relative cumulative lighting time period increases. Therefore, it is observed that when the cumulative lighting time period increases, the light emission quantity of the first LED 14 decreases at a higher degradation rate than that of the second LED 15.

When the first and second degradation rates are different as shown in Fig. 3, there is a difference in the degradation level between the first LED 14 and the second LED 15 except in the case of an accident where the first and second degradation rates complementarily match in the degradation rate and the cumulative lighting time period (e.g., the first degradation rate is -10 (%/1000 hours) and the second degradation rate is -5 (%/1000 hours), but since the cumulative lighting time period of the first LED 14 is 500 hours and the cumulative lighting time period of the second LED 15 is 1000 hours, both LEDs have an identical degradation level of -5%).

Furthermore, even when the first degradation rate is identical to the second degradation rate, if the cumulative lighting time period of the first LED 14 is different from the cumulative lighting time period of the second LED 15, there arises a difference in the degradation level.

For example, suppose the light source apparatus 1 is provided with an R-LED that emits red light, a G-LED that emits green light and a B-LED that emits blue light. Suppose the image pickup system is allowed to have a white light observation mode carried out by turning on the R-LED, the G-LED and the B-LED and a fluorescence observation mode carried out by turning on the G-LED as a reference light emission source and the B-LED as an excitation light emission source. In such a case, the G-LED and the B-LED emit light in both the white light observation mode and the fluorescence observation mode, whereas the R-LED emits light only in the white light observation mode and does not emit light in the fluorescence observation mode. Therefore, there can be a difference in the cumulative lighting time period among the R-LED, the G-LED and the B-LED, resulting in a difference in the degradation level as well.

In addition, suppose the light source apparatus 1 is provided with, for example, a V-LED for emitting violet light in addition to the R-LED, the G-LED and the BLED. Suppose the image pickup system is further allowed to have an NBI observation mode carried out by turning on the V-LED as a first narrow-band light emission light source and the G-LED as a second narrow-band light emission light source. An example of aspects of light emission in such a case is an aspect in which the R-LED emits light only in the white light observation mode, the V-LED emits light only in the NBI observation mode, the B-LED emits light in the white light observation mode and the fluorescence observation mode and the G-LED emits light in the white light observation mode, the fluorescence observation mode and in the NBI observation mode (however, these are only examples because there is an aspect in which the B-LED and the V-LED emit light simultaneously in the white light observation mode). Thus, there can be a difference in the cumulative lighting time period among the R-LED, the G-LED, the B-LED and the V-LED, that is, there can also be a difference in the degradation level.

The filter 16 is configured as, for example, a beam splitter to reflect the first-band light emitted from the first LED 14, guide the reflected first-band light to an incident end of the light guide 21 and transmit the second-band light emitted from the second LED 15 and guide the transmitted second-band light to the incident end of the light guide 21.

The video signal processing section 17 is configured to process a video signal acquired from the image pickup device 22 and output the processed video signal to the display apparatus 3. As shown in Fig. 2, the video signal processing section 17 is provided with a light-emitting element degradation level correction section 18 which is a degradation level correction section and a color balance correction section 19 which is provided after the light-emitting element degradation level correction section 18. Here, Fig. 2 is a block diagram illustrating a configuration of the video signal processing section 17.

The light-emitting element degradation level correction section 18 acquires a first light emission quantity which is the current light emission quantity of the first LED 14 based on the first cumulative lighting time period read from the storage section 13 and acquires a second light emission quantity which is the current light emission quantity of the second LED 15 based on the second cumulative lighting time period read from the storage section 13.

To be more specific, the light-emitting element degradation level correction section 18 calculates a first reduction rate ($V1 \times t1$) by multiplying a first degradation rate V1 of the first LED 14 by a first cumulative lighting time period t1 which is a cumulative lighting time period of the first LED 14 at the current point in time, and calculates a first light emission quantity A1(t1) at the first cumulative lighting time period t1, by multiplying the value ($1-V1 \times t1$) obtained by subtracting the first reduction rate from 1 by the first initial light emission quantity A1(0) as:

$$A1(t1) = (1 - V1 \times t1) \times A1(0)$$

Furthermore, the light-emitting element degradation level correction section 18 calculates a second reduction rate ($V2 \times t2$) by multiplying the second degradation rate V2 of the second LED 15 by a second cumulative lighting time period t2 which is a cumulative lighting time period of the second LED 15 at the current point in time and calculates a second light emission quantity A2(t2) at the second cumulative lighting time period t2 by multiplying the value ($1-V2 \times t2$) obtained by subtracting the second reduction rate from 1 by the second initial light emission quantity A2(0) as:

$$A2(t2) = (1 - V2 \times t2) \times A2(0)$$

Note that such an approximation has been made above that the first degradation rate V1 of the first LED 14 and the second degradation rate V2 of the second LED 15 take constant values (that is, an approximation in which the first degradation rate V1 and the second degradation rate V2 deteriorate at a constant rate as the cumulative lighting time period increases), but more specifically, the light emission quantity A(t) at the cumulative lighting time period t may vary using the initial light emission quantity A(0) and the positive degradation coefficient $\alpha$, for example:

$$A(t) = A(0)\exp\{-(\alpha \times t)\}$$

Thus, using this equation, the first light emission quantity A1 and the second light emission quantity A2 may be calculated as:

$$A1(t1) = A1(0)\exp\{-(\alpha 1 \times t1)\}$$

$$A2(t2) = A2(0)\exp\{-(\alpha 2 \times t2)\}$$

where $\alpha 1$ is a positive degradation coefficient associated with the first LED 14 and $\alpha 2$ is a positive degradation coefficient associated with the second LED 15. Note that a variation in the degradation level of the light-emitting element may be described using a more complicated equation.

Furthermore, when table reference is used instead of calculation, the light-emitting element degradation level correction section 18 refers to the aforementioned first table using the first cumulative lighting time period to thereby acquire a first light emission quantity, and refers to the aforementioned second table using the second cumulative lighting time period to thereby acquire a second light emission quantity.

The light-emitting element degradation level correction section 18 calculates a gain coefficient by dividing one of the first light emission quantity A1 (t1) and the second light emission quantity A2(t2) by the other, performs gain adjustment using the calculated gain coefficient on the other of the first video signal and the second video signal, and thereby corrects a difference in a degradation level between the first LED 14 and the second LED 15.

For example, the light-emitting element degradation level correction section 18 calculates a numeric value A1(t1)/A2(t2) obtained by dividing the first light emission quantity A1 (t1) by the second light emission quantity A2(t2) as a gain coefficient and performs gain adjustment using the calculated gain coefficient on a second video signal Sig2 obtained by light emission of the second LED 15 to calculate a corrected second video signal Sig2' as:

$$Sig2' = Sig2 \times A1(t1)/A2(t2)$$

to correct a difference in a degradation level between the first LED 14 and the second LED 15.

By correcting the video signal in such a way, it is possible to obtain a video signal when the light emission quantity attenuation ratio due to a change over time becomes substantially equal for each color LED as shown in Fig. 4, that is, a video signal substantially equal to that when the color balance is in an initial state (cumulative lighting time period = 0). Here, Fig. 4 is a time chart illustrating an example where the light emission quantity attenuation ratio due to a change over time becomes substantially equal for each color LED.

Note that gain adjustment is performed here by adjusting the second video signal to the first video signal, but instead of this, the first video signal Sig1 obtained by light emission of the first LED 14 may be corrected as:

$$Sig1' = Sig1 \times A2(t2)/A1(t1)$$

to calculate a corrected first video signal Sig1' to correct the difference in a degradation level between the first LED 14 and the second LED 15. Thus, a video signal serving as a reference (by extension, light-emitting element serving as a reference) can be determined as appropriate.

That is, since each light-emitting element has rated power that can be supplied, it is difficult to supply large power to complement the degradation level to a light-emitting element that has a large degradation level (e.g., first LED 14) so as to cause the light-emitting element to emit light with the same light emission quantity as that in the initial state. Therefore, there is no choice but to adjust a light-emitting element at a lower degradation level to a light-emitting element at a higher degradation level and decrease the light emission quantity of the light-emitting element at the lower degradation level. In contrast, degradation level correction according to the present embodiment is multiplication of a video signal by a gain coefficient in the video signal processing section 17, and it is thereby possible to increase the gain of the video signal obtained by causing the light-emitting element at the higher degradation level to emit light so as to adjust the video signal obtained by causing the light-emitting element at the lower degradation level to emit light, and has an advantage that it is possible to obtain a signal with substantially the same brightness as that when light is emitted in an initial state.

The color balance correction section 19 performs color balance adjustment on the first video signal and the second video signal corrected by the light-emitting element degradation level correction section 18. That is, since the first video signal and the second video signal on which the color balance correction section 19 performs color balance adjustment are video signals whose difference in the degradation level is corrected by the light-emitting element degradation level correction section 18, it is possible to perform normal color balance adjustment similar to color balance adjustment on video signals without difference in the degradation level. Since the processing by the light-emitting element degradation level correction section 18 is performed before the color balance correction section 19, the color balance correction section 19 need not be provided with a special component different from the conventional one and has an advantage that it is possible to perform processing similar to that when processing an image obtained by picking up an image of an object illuminated with well color-balanced illuminating light similar to that in the initial state.

Such Embodiment 1 acquires light emission quantities of a plurality of light-emitting elements in different bands based on a cumulative lighting time period of each light-emitting element and performs gain adjustment on video signals based on the ratio of light emission quantities, and it is thereby possible to correct a difference in the degradation level among a plurality of light-emitting elements and prevent, at low cost, the color balance of illuminating light from changing with time.

Furthermore, when initial light emission quantities and degradation rates of a plurality of light-emitting elements in different bands are stored in advance and the current light emission quantity is calculated using the cumulative lighting time period, the storage section 13 needs only to have a small storage capacity and the calculation itself is simple, providing an advantage that the burden of processing is light.

On the other hand, when the correspondence between a cumulative lighting time period and a light emission quantity is stored as a table, even when the relationship between the cumulative lighting time period and the light emission quantity is complicated when described using a function, since table reference is used, if information on the cumulative lighting time period is available, the current light emission quantity can be easily acquired.

Furthermore, an image pickup system capable of performing white light observation and fluorescence observation can appropriately correct a difference in the degradation level among the R-LED, the G-LED or the B-LED where there may be a difference in a cumulative lighting time period.

An image pickup system capable of performing NBI observation in addition to white light observation and fluorescence observation can appropriately correct a difference in the degradation level among the R-LED, the G-LED, the B-LED or the V-LED where there may be a difference in a cumulative lighting time period.

Since the color balance correction section 19 performs color balance adjustment on the video signal corrected by the light-emitting element degradation level correction section 18, the color balance correction section 19 can perform color balance adjustment similar to that of the prior art without taking the degradation levels of light-emitting elements into consideration.

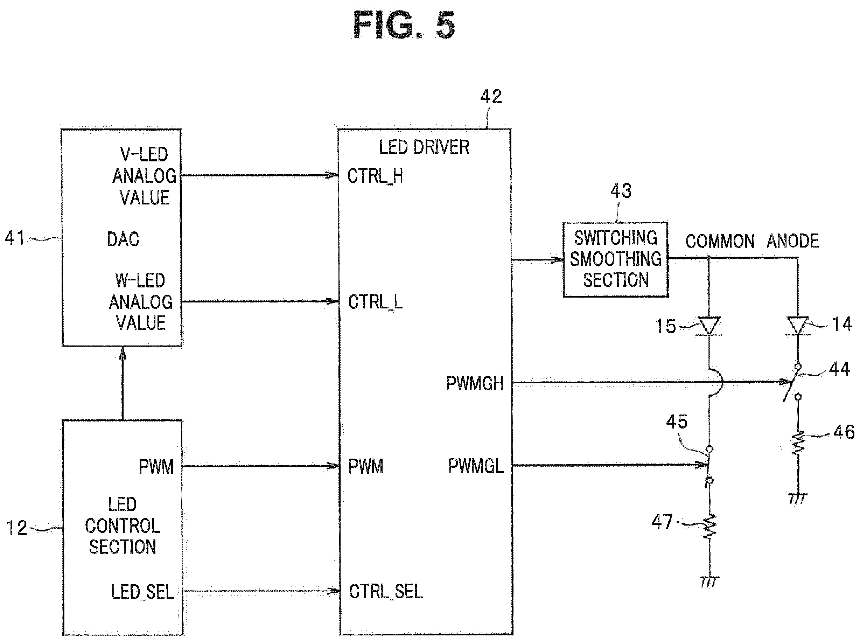

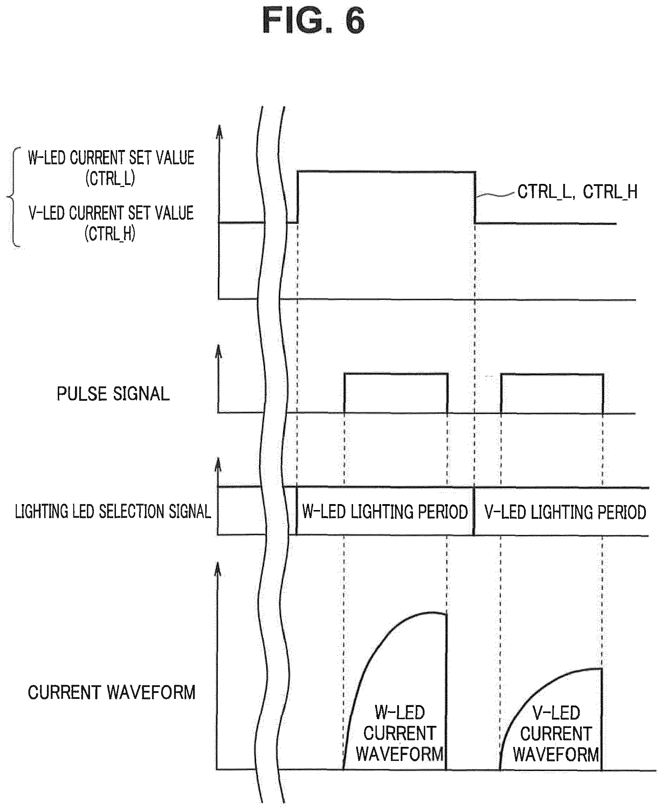

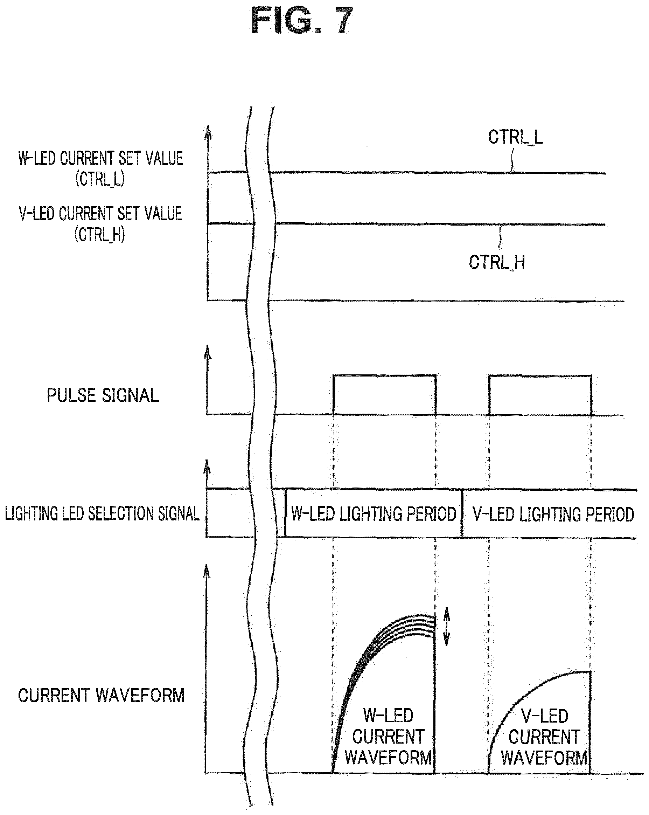

Next, the configuration related to the aforementioned embodiment will be described with reference to Fig. 5 to Fig. 7. Fig. 5 is a diagram illustrating a configuration of an LED drive circuit, Fig. 6 is a timing chart illustrating an example of a signal flowing through the LED drive circuit in Fig. 5 and Fig. 7 is a timing chart illustrating an example where the current waveform becomes unstable when one LED driver causes a plurality of LEDs to emit light.

When the LED control section 12 drives the first LED 14 and the second LED 15, one LED driver (light-emitting element drive circuit) may be used concurrently as the LED driver to drive the first LED 14 and as the LED driver to drive the second LED 15 for the purpose of reducing the price. Fig. 5 shows an example of such a configuration.

As shown in Fig. 5, the LED control section 12 is configured to drive the first LED 14 (e.g., V-LED (violet LED)) and the second LED 15 (e.g., W-LED (white LED)) using one LED driver 42.

That is, a DAC (digital/analog converter) 41 and an LED driver 42 are connected to the LED control section 12 so that a digital signal is outputted to the LED driver 42 and an analog signal is outputted to the LED driver 42 via the DAC 41.

The LED driver 42 is further connected to a switching smoothing section 43 and connected to a switch 44 and a switch 45 via a PWMGH terminal and a PWMGL terminal respectively.

The switching smoothing section 43 is grounded via the first LED 14, the switch 44 and a resistor 46, and further grounded via the second LED 15, the switch 45 and a resistor 47. The first LED 14 and the second LED 15 are connected to the switching smoothing section 43 via a common anode.

The LED control section 12 outputs a digital signal indicating a W-LED current set value and a digital signal indicating a V-LED current set value to the DAC 41.

The DAC 41 converts a digital signal to an analog signal and outputs a W-LED analog value signal (current set value CTRL_L) and a V-LED analog value signal (current set value CTRL_H) to a CTRL_L input terminal of the LED driver 42 and a CTRL_H input terminal of the LED driver 42 respectively (also see Fig. 6 and Fig. 7).

A lighting LED selection signal is inputted to a CTRL_SEL terminal of the LED driver 42 from an LED_SEL terminal of the LED control section 12. The lighting LED selection signal becomes a signal for selecting the current set value CTRL_L to turn on the switch 45 and turn off the switch 44 during a W-LED lighting period of, for example, a lighting period which is sequentially rotated and becomes a signal for selecting the current set value CTRL_H to turn on the switch 44 and turn off the switch 45 during a V-LED lighting period (also see Fig. 6 and Fig. 7).

Furthermore, a pulse signal PWM which is a pulse width modulation signal is inputted to a PWM terminal of the LED driver 42 from a PWM terminal of the LED control section 12 (also see Fig. 6 and Fig. 7).

Upon receiving the pulse signal PWM during the W-LED lighting period, the LED driver 42 sets a W-LED current value and outputs a pulse-width-modulated drive pulse to the switching smoothing section 43.

The switching smoothing section 43 smooths the drive pulse inputted from the LED driver 42 using a capacitor or the like. Thus, the smoothed W-LED current is applied to the second LED 15 which is a W-LED (also see Fig. 6 and Fig. 7).

Upon receiving the pulse signal PWM during the V-LED lighting period, the LED driver 42 sets a V-LED current value and outputs a pulse-width-modulated drive pulse to the switching smoothing section 43.

The switching smoothing section 43 smooths the drive pulses inputted from the LED driver 42 as described above. Thus, the smoothed V-LED current is applied to the first LED 14 which is a V-LED (also see Fig. 6 and Fig. 7).

In such a configuration, when the current set value CTRL_L set in the LED driver 42 is different from the current set value CTRL_H (particularly when the difference in the current set value is large), the LED driver 42 may not stably operate because the one current set value is affected by the other current set value depending on the circuit characteristic of the LED driver 42.

For example, as shown in Fig. 7, when the current set value CTRL_L for the W-LED is larger than the current set value CTRL_H for the V-LED (CTRL_L>CTRL_H), the current waveform applied to the second LED 15 which is the W-LED may be unstable.

Thus, an example of control to handle such a case will be described with reference to Fig. 6.

In the control shown in Fig. 7, the LED control section 12 outputs a digital signal indicating the V-LED current set value and a digital signal indicating the W-LED current set value to the DAC 41 as constant values which do not change over time.

In contrast, in the control shown in Fig. 6, the LED control section 12 equalizes the digital signal indicating the V-LED current set value and the digital signal indicating the W-LED current set value, causes both digital signals to change over time so that both digital signals become different set values for the W-LED lighting period and the V-LED lighting period, and outputs the set values to the DAC 41.

That is, the LED control section 12 outputs a digital signal to the DAC 41 so that the same value as that of CTRL_L shown in Fig. 7 becomes the CTRL_L and CTRL_H values in Fig. 6 for the W-LED lighting period and outputs a digital signal to the DAC 41 so that the same value as that of CTRL_H shown in Fig. 7 becomes the CTRL_L and CTRL_H values in Fig. 6 for the V-LED lighting period.

In this way, although the current set value CTRL_L and the current set value CTRL_H inputted to the LED driver 42 change over time, both current set values have the same value when seen at the same point in time. Therefore, there is no difference between the current set value CTRL_L and the current set value CTRL_H at any given time, and it is possible to stabilize the current waveform applied to the first LED 14 and the second LED 15.

Note that although two light-emitting elements are driven by one light-emitting element drive circuit above, the aforementioned technique is likewise applicable to a case where three or more light-emitting elements are driven by one light-emitting element drive circuit.

According to the configuration associated with the above embodiment, when a plurality of light-emitting elements are driven by one light-emitting element drive circuit, it is possible to stabilize a current waveform to be outputted to any light-emitting element and stabilize light emission luminance of each light-emitting element. Thus, it is possible to keep the color balance of a video signal constant and improve the video quality.

Note that the present invention is not limited to the aforementioned embodiment as is, but the components of the invention can be modified and implemented without departing from the spirit and scope of the invention at an implementation phase. Furthermore, various aspects of the invention can be formed by an appropriate combination of the plurality of components disclosed in the above-described embodiment. For example, several components may be deleted from all the components shown in the embodiment. Moreover, components across different embodiments may be combined as appropriate. Thus, it goes without saying that various modifications and applications are possible without departing from the spirit and scope of the present invention.

The present application claims priority based on Japanese Patent Application No. 2014-100797, filed in Japan on May 14, 2014, the disclosure of which is incorporated in the specification, claims and drawings of the present application by reference.

**Claims**

1. An image pickup system that irradiates an object with light and picks up an image of the object, the image pickup system comprising:

    a first light-emitting element configured to turn on when power is supplied to emit first-band light, a first light emission quantity of which decreases at a first degradation rate as a first cumulative lighting time period increases;
    a second light-emitting element configured to turn on when power is supplied to emit second-band light, a second light emission quantity of which decreases at a second degradation rate as a second cumulative lighting time period increases;
    an image pickup section configured to pick up an optical image of the object irradiated with the light from the first light-emitting element and the second light-emitting element and generate a first video signal associated with the first-band light and a second video signal associated with the second-band light; and
    a degradation level correction section configured to acquire the first light emission quantity based on the first cumulative lighting time period, acquire the second light emission quantity based on the second cumulative lighting time period, calculate a gain coefficient by dividing one of the first light emission quantity and the second light emission quantity by another, perform gain adjustment using the calculated gain coefficient on another of the first video signal and the second video signal and thereby correct a difference in a degradation level between the first light-emitting element and the second light-emitting element.

2. The image pickup system according to claim 1, further comprising a storage section configured to store the first degradation rate, the second degradation rate, a first initial light emission quantity of the first light-emitting element in an initial state and a second initial light emission quantity of the second light-emitting element in an initial state,
   wherein the degradation level correction section calculates a first reduction rate by multiplying the first degradation rate by the first cumulative lighting time period and calculates the first light emission quantity by multiplying a value obtained by subtracting the first reduction rate from 1 by the first initial light emission quantity, and
   calculates a second reduction rate by multiplying the second degradation rate by the second cumulative lighting time period and calculates the second light emission quantity by multiplying a value obtained by subtracting the second reduction rate from 1 by the second initial light emission quantity.

3. The image pickup system according to claim 1, further comprising a storage section configured to store a first table to indicate correspondence between the first cumulative lighting time period corresponding to the first degradation rate and the first light emission quantity and a second table to indicate correspondence between the second cumulative lighting time period corresponding to the second degradation rate and the second light emission quantity,
   wherein the degradation level correction section acquires the first light emission quantity by referring to the first table using the first cumulative lighting time period and acquires the second light emission quantity by referring to the second table using the second cumulative lighting time period.

4. The image pickup system according to claim 1, further comprising an R-LED configured to emit red light, a G-LED configured to emit green light and a BLED configured to emit blue light,
   wherein the image pickup system can perform white light observation by turning on the R-LED, the G-LED and the B-LED, and fluorescence observation by turning on the G-LED as a reference light emission source and by turning on the BLED as an excitation light emission source, and
   the first light-emitting element is the R-LED and the second light-emitting element is the G-LED or the B-LED.

5. The image pickup system according to claim 4, further comprising a V-LED configured to emit violet light,
   wherein the image pickup system can further perform NBI observation by turning on the V-LED as a first narrow-band light emission light source and the G-LED as a second narrow-band light emission light source, and
   the second light-emitting element is the G-LED, the B-LED or the V-LED.

6. The image pickup system according to claim 1, further comprising a color balance correction section configured to perform color balance adjustment on the first video signal and the second video signal corrected by the degradation level correction section.

EP 3 146 889 A1

# FIG. 1

LIGHT SOURCE APPARATUS 1

FIRST LED — 14

16:FILTER

2:SCOPE 21

LED CONTROL SECTION (11, 12)

SECOND LED (15)

LIGHT GUIDE

CONTROL SECTION

STORAGE SECTION (13)

VIDEO SIGNAL PROCESSING SECTION (17)

22:IMAGE PICKUP DEVICE

DISPLAY APPARATUS — 3

# FIG. 2

VIDEO SIGNAL PROCESSING SECTION 17

FROM STORAGE SECTION

COLOR BALANCE CORRECTION SECTION (19)

LIGHT-EMITTING ELEMENT DEGRADATION LEVEL CORRECTION SECTION (18)

FROM IMAGE PICKUP DEVICE

TO DISPLAY APPARATUS

10

# FIG. 3

# FIG. 4

# FIG. 5

EP 3 146 889 A1

# FIG. 6

W-LED CURRENT SET VALUE
(CTRL_L)

V-LED CURRENT SET VALUE
(CTRL_H)

CTRL_L, CTRL_H

PULSE SIGNAL

LIGHTING LED SELECTION SIGNAL

W-LED LIGHTING PERIOD | V-LED LIGHTING PERIOD

CURRENT WAVEFORM

W-LED
CURRENT
WAVEFORM

V-LED
CURRENT
WAVEFORM

EP 3 146 889 A1

# FIG. 7

W-LED CURRENT SET VALUE (CTRL_L)

CTRL_L

V-LED CURRENT SET VALUE (CTRL_H)

CTRL_H

PULSE SIGNAL

LIGHTING LED SELECTION SIGNAL | W-LED LIGHTING PERIOD | V-LED LIGHTING PERIOD

CURRENT WAVEFORM

W-LED CURRENT WAVEFORM

V-LED CURRENT WAVEFORM

14

## INTERNATIONAL SEARCH REPORT

| International application No. |
| --- |
| PCT/JP2015/059479 |

A. CLASSIFICATION OF SUBJECT MATTER
*A61B1/06*(2006.01)i, *A61B1/00*(2006.01)i, *A61B1/04*(2006.01)i, *F21S2/00*
(2006.01)i, *G03B7/16*(2014.01)i, *G03B15/02*(2006.01)i, *G03B15/05*(2006.01)i,
*H04N9/04*(2006.01)i, *H05B37/02*(2006.01)i, *F21Y101/02*(2006.01)n
According to International Patent Classification (IPC) or to both national classification and IPC

B. FIELDS SEARCHED

Minimum documentation searched (classification system followed by classification symbols)
A61B1/06, A61B1/00, A61B1/04, F21S2/00, G03B7/16, G03B15/02, G03B15/05,
H04N9/04, H05B37/02, F21Y101/02

Documentation searched other than minimum documentation to the extent that such documents are included in the fields searched
Jitsuyo Shinan Koho          1922–1996   Jitsuyo Shinan Toroku Koho   1996–2015
Kokai Jitsuyo Shinan Koho    1971–2015   Toroku Jitsuyo Shinan Koho   1994–2015

Electronic data base consulted during the international search (name of data base and, where practicable, search terms used)
JSTPlus/JMEDPlus/JST7580(JDreamIII)

C. DOCUMENTS CONSIDERED TO BE RELEVANT

| Category* | Citation of document, with indication, where appropriate, of the relevant passages | Relevant to claim No. |
| --- | --- | --- |
| A | JP 2008-243584 A  (Matsushita Electric Works, Ltd.), 09 October 2008 (09.10.2008), paragraphs [0008], [0011] to [0012], [0024] (Family: none) | 1-6 |
| A | WO 2012/161028 A1  (Olympus Medical Systems Corp.), 29 November 2012 (29.11.2012), paragraphs [0002] to [0007], [0033] to [0064] & US 2013/0154509 A1     & EP 2604178 A1 & CN 103153165 A          & JP 5198693 B2 | 1-6 |

| ☒ | Further documents are listed in the continuation of Box C. | ☐ | See patent family annex. |

\* Special categories of cited documents:
"A" document defining the general state of the art which is not considered   to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed

"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
| --- | --- |
| 05 June 2015 (05.06.15) | 16 June 2015 (16.06.15) |

| Name and mailing address of the ISA/ Japan Patent Office 3-4-3,Kasumigaseki,Chiyoda-ku, Tokyo 100-8915,Japan | Authorized officer<br><br>Telephone No. |

Form PCT/ISA/210 (second sheet) (July 2009)

## INTERNATIONAL SEARCH REPORT

| International application No. |
|---|
| PCT/JP2015/059479 |

C (Continuation). DOCUMENTS CONSIDERED TO BE RELEVANT

| Category* | Citation of document, with indication, where appropriate, of the relevant passages | Relevant to claim No. |
|---|---|---|
| A | WO 2010/041494 A1 (Sharp Corp.),<br>15 April 2010 (15.04.2010),<br>paragraphs [0010] to [0011]<br>& US 2011/0175950 A1 & EP 2334152 A1<br>& CN 102138366 A & RU 2011113160 A<br>& JP 5037695 B2 | 1-6 |
| A | JP 2011-233263 A (Panasonic Electric Works Co.,<br>Ltd.),<br>17 November 2011 (17.11.2011),<br>paragraphs [0013] to [0018]<br>(Family: none) | 1-6 |
| A | JP 2009-201940 A (Hoya Corp.),<br>10 September 2009 (10.09.2009),<br>paragraphs [0007] to [0009], [0027], [0034],<br>[0038]<br>& US 2009/0221875 A1 & DE 102009011227 A1 | 1-6 |

Form PCT/ISA/210 (continuation of second sheet) (July 2009)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2012032619 A
- JP 2011233263 A
- WO 2010041494 A
- JP 2014100797 A